# EUROPEAN PATENT SPECIFICATION

(11) **EP 2 441 733 B1**
(45) Date of publication and mention of the grant of the patent: **31.12.2014**
(21) Application number: 11185032.7
(22) Date of filing: 13.10.2011
(51) Int. Cl.: C01B 39/02, B01J 29/06, C07C 5/22, C07C 4/06

(54) **A NU-85 molecular sieve having a large pore volume and processes for preparing the same**
NU-85-Molekülsieb mit einer hohen Porenmenge und Herstellungsverfahren dafür
Tamis moléculaire NU-85 doté d'un grand volume de pores et procédés de préparation

(30) Priority: 13.10.2010 CN 201010509203
(43) Date of publication of application: 18.04.2012
(73) Proprietor: China Petroleum & Chemical Corporation, Beijing 100728 (CN); Fushun Research Institute of Petroleum and Petrochemicals, Sinopec, Wanghua District, Fushun 113001 Liaoning Province (CN)
(72) Inventor: Zhang, Zhizhi, Fushun Liaoning Province 113001 (CN); Zhang, Xiwen, Fushun Liaoning Province 113001 (CN); Qin, Bo, Fushun Liaoning Province 113001 (CN)
(74) Representative: Hoffmann Eitle

(56) References cited:
- EP-A2- 0 462 745
- WO-A1-2008/152214
- FR-A1- 2 765 207
- FR-A1- 2 775 483

## Description

### Technical field

The present disclosure relates to a novel NU-85 molecular sieve and a process for preparing the same.

### Background art

NU-85 molecular sieves are a high-silicon zeolite molecular sieve having a two-dimensional micropore structure. NU-85 molecular sieves are the intergrowth of EU-1 molecular sieves and NU-87 molecular sieves, and the structural bands of EU-1 molecular sieves and NU-87 molecular sieves therein are interwoven and tightly bound. With such a structure, NU-85 molecular sieves can have the pore channel features and catalytic properties of the two parent molecular sieves, which also enable them to be widely used as the catalyst in the isomerization and alkylation of hydrocarbon compounds.

US5385718 and US6784334 firstly disclose some NU-85 molecular sieves and processes for preparing the same, wherein the template used therein is hexamethonium bromide (HexBr₂), and the synthesis thereof is carried out by hydrothermal crystallization. The specific synthetic process is as follows: dissolving an aluminum source, a base and an inorganic salt in a part of water to form a solution A, dissolving a template in a part of water to form a solution B, dissolving an silicon source in water to form a solution C, firstly adding the solution B into the solution A with homogeneously stirring, then adding the solution C into the solution A with homogeneously stirring. The materials added therein have a molar ratio of SiO₂/Al₂O₃=25-40, Na₂O/SiO₂=0.025-0.5, R/SiO₂=0.02-1, H₂O/SiO₂=5-250 and NaL/SiO₂=0-1, wherein R is a template hexamethonium bromide; NaL is a sodium salt. The crystallization temperature is from 120 to 180°C, and the crystallization time is more than 10 days. Crystal seeds may be added to shorten the crystallization time, wherein the crystal seeds added may be NU-85 molecular sieves, NU-87 molecular sieves, and EU-1 molecular sieves. The NU-85 molecular sieves synthesized under different conditions have different ratios of EU-1 molecular sieves to NU-87 molecular sieves. Temperature has a great influence on the synthesis of NU-85 molecular sieves. When the crystallization temperature is 200°C, the synthesized product is the EU-1 molecular sieve; when the crystallization temperature is 180°C, the EU-1 molecular sieve is preferably produced, but the product has exhibited the features of the NU-85 molecular sieves; when the crystallization temperature is 160°C, the synthesized product is the NU-85 molecular sieve. Along with the decrease of the crystallization temperature, the product will change from pure EU-1 molecular sieves to the NU-85 molecular sieves intergrowed by EU-1 molecular sieves and NU-87 molecular sieves. However, the lower the temperature is, the longer the crystallization time is. Generally, the NU-85 molecular sieves have a crystallization time of more than 18 days.

NU-85 molecular sieves generally have the XRD characteristic spectral bands shown in the following Table as follows

The XRD characteristic spectral bands of NU-85 molecular sieves

| Peak No. | 2θ | d | Relative intensity |
|---|---|---|---|
| 1 | 7.96 | 11.09 | Medium |
| 2 | 8.80 | 10.2 | Weak^{a} |
| 3 | 19.05 | 4.65 | Medium |
| 4 | 20.56 | 4.31 | 100 |
| 5 | 22.18 | 4.00 | Strong-very strong |
| 6 | 23.27 | 3.81 | Weak-medium |
| 7 | 23.97 | 3.70 | Medium ^{b} |
| 8 | 25.99 | 3.42 | Weak-medium |
| 9 | 26.53 | 3.35 | Weak-medium^{c} |
| 10 | 27.3 | 3.26 | Strong-very strong |

On the basis of such relative intensity gauge, the strongest relative intensity value is defined as 100; the weak represents a value less than 20; the medium represents a value of 20-40; the strong represents a value of 40-60; the very strong represents a value of more than 60. In the table above, the intensity ratio of the characteristic spectral line marked by "a" to the previous characteristic spectral line is not greater than 0.5; the intensity ratio of the characteristic spectral line marked by "b" to the latter characteristic spectral line is not greater than 1.0; the intensity ratio of the characteristic spectral line marked by "c" to the latter characteristic spectral line is not greater than 1.0.

The NU-85 molecular sieves prepared according to the aforesaid process have a small pore volume ranging generally less than 0.20 cm³/g and a small specific surface area ranging generally less than 400 m²/g.

### Contents of the invention

Disclosed herein is a process for preparing an NU-85 molecular sieve, which can effectively shorten the crystallization time of NU-85 molecular sieves. The resultant NU-85 molecular sieve has large specific surface area and pore volume.

The NU-85 molecular sieves of the present invention have a specific surface area ranging from about 405 m²/g to about 470 m²/g, preferably from about 410 m²/g to about 460 m²/g, and a pore volume ranging from about 0.27 cm³/g to about 0.35 cm³/g, preferably from about 0.28 cm³/g to about 0.32 cm³/g.

The process for preparing the NU-85 molecular sieve of the present invention comprises the formulating procedure and the crystallization procedure of the crystallization material, and the recovery procedure of the crystallization product, wherein the formulating procedure of the crystallization material comprises mixing an aluminum source, a silicon source, a base, a template, an inorganic salt, EU-1 molecular sieve and water in a mass ratio of the EU-1 molecular sieve to SiO₂ in the silicon source ranging from about 2.5 to about 8.5, preferably from about 3.0 to about 7.0.

The molar ratio of the aluminum source (calculated based on Al₂O₃), silicon source (calculated based on SiO₂), base (calculated based on Na₂O), template, inorganic salt (calculated based on NaL) and water in the crystallization material are:
SiO₂/Al₂O₃=about 30:1 to about 70:1;
R/SiO₂=about 0.04:1 to about 0.25:1;
Na₂O/SiO₂=about 0.05:1 to about 0.3:1;
H₂O/SiO₂=about 35:1 to about 80:1;
NaL/SiO₂=about 0.03:1 to about 0.14:1;
   preferably:
SiO₂/Al₂O₃=about 40:1 to about 65:1;
R/SiO₂=about 0.08:1 to about 0.22:1;
Na₂O/SiO₂=about 0.1:1 to about 0.2:1;
H₂O/SiO₂=about 4.2:1 to about 55:1;
NaL/SiO₂=about 0.049:1 to about 0.098:1;
wherein R is a template selected from the group consisting of nonamethonium bromide, decamethonium bromide, and undecamethonium bromide, or mixture thereof, preferably decamethonium bromide; NaL is a sodium salt. Said sodium salt may be selected from the group consisting of sodium chloride, sodium bromide, sodium nitrate, sodium acetate and sodium oxalate, or mixture thereof.In some embodiments, NaL is sodium chloride.

The crystallizing procedure of the formulated crystallization material comprises dynamic crystallization which comprises two stages: the low-temperature crystallization and the high-temperatrue crystallization, wherein the low-temperature crystallization is firstly carried out by a high-speed stirring at a stirring rate ranging from about 300 rpm to about 800 rpm and at a crystallization temperature ranging from about 100°C to about 160°C for a period of time ranging from about 1 day to about 2 days; then the high-temperature crystallization is carried out by a low-speed stirring at a stirring rate ranging from about 40 rpm to about 250 rpm and at a crystallization temperature ranging from about 170°C to about 190°C for a period of time ranging from about 2 days to about 6 days. The entire process of dyanmic crystallizing procedure is carried out under autogenous pressure at the crystallization temperature.

The aluminum source is one or more selected from the group consisting of aluminum salts, aluminates, alumina, aluminum hydroxide and alumina sols, or mixtures thereof, preferably sodium aluminate; the silicon source is selected from the group consisting of white carbon black, silica sol, water glass and tetraethyl orthosilicate, or mixtures thereof, preferably silica sol. The base used herein comprises NaOH, etc.

The SiO₂/Al₂O₃ molar ratio in the EU-1 molecular sieve is from about 20:1 to about 35:1. The EU-1 molecular sieve may be calcined, or non-calcined EU-1 molecular sieve, preferably the calcined EU-1 molecular sieve.

The NU-85 molecular sieve prepared according to the present invention can be applied in the fields such as petrochemical industry and can serve as catalytic materials. Such molecular sieves can have good catalytic performance in aromatic alkylation, aromatic isomerization, alkane hydroisomerization, and aromatization of lighter hydrocarbons such as C 1-8 alkanes and alkenes.

In the process of the present invention, the EU-1 molecular sieve is applied in the formulating procedure of the crystallization material. The EU-1 molecular sieve is a molecular sieve having an extremely similar structure to that of the NU-85 molecular sieve, and has the features of nano-crystals at the same time. The process of the present invention sufficiently utilizes the features of nano-crystals of the Euro-type molecular sieve to decompose it during the formulating procedure of the crystallization material and to form a plurality of secondary structural units of the EUO-type molecular sieve, wherein these structural units are also the structural units of the resultant NU-85 molecular sieves. These secondary structural units are used as the directing agent for incorporating silicon, aluminium into the framework of the NU-85 molecular sieve. The process of the present invention effectively shortens the crystallization time of the NU-85 molecular sieves, reduces the preparation cost and increases the production efficiency. Although nonamethonium bromide, decamethonium bromide, and undecamethonium bromide are not the conventional template for synthesizing the NU-85 molecular sieves, they can effectively promote the synthesis of the NU-85 molecular sieves by the process of the present invention, so as to achieve unexpected technical effect. The present invention comprises two-phase dynamic crystallization, and the resultant product has a large pore volume and specific surface area.

The NU-85 molecular sieves prepared according to the process of the present invention can be used for the C8 aromatic isomerization, toluene disproportionation and trimethylbenzene trans-alkylation reaction, as well as for the preparation of ethyl benzene using benzene and ethylene, and have better catalytic effect.

### Description of the Figures

Fig. 1 shows the X-ray diffraction (XRD) patterns of the NU-85 molecular sieve prepared according to Example 1 of the present invention.
Fig. 2 shows the scanning electron microscope figure of the NU-85 molecular sieve prepared according to Example 1 of the present invention.

### Embodiment

The present invention is further illustrated by the following examples, but is not limited by these examples.

The specific surface area of the NU-85 molecular sieves of the present invention was measured according to the ASTM D3663-2003 method; the pore volume thereof was measured according to the ASTM D4222-2003 method.

### Example 1

Formulating solution 1: 2.61 g of NaAlO₂, 4.37 g of NaOH, 4.26 g of NaCl and 60.32 g of DecBr₂ (decamethonium bromide) were added into 300 ml of H₂O, and homogeneously mixed with stirring.

Formulating solution 2: 125 ml of silica sol (containing 45 g of SiO_{2,} the same below) was added into 267 ml of H₂O, and homogeneously mixed with stirring.

The solution 2 was added into the solution 1, and homogeneously mixed with stirring. 125 g of EU-1 molecular sieve (prepared according to the process of Example 1 in WO 2008/152214, the same below) was added as the crystal seed to prepare a gel.

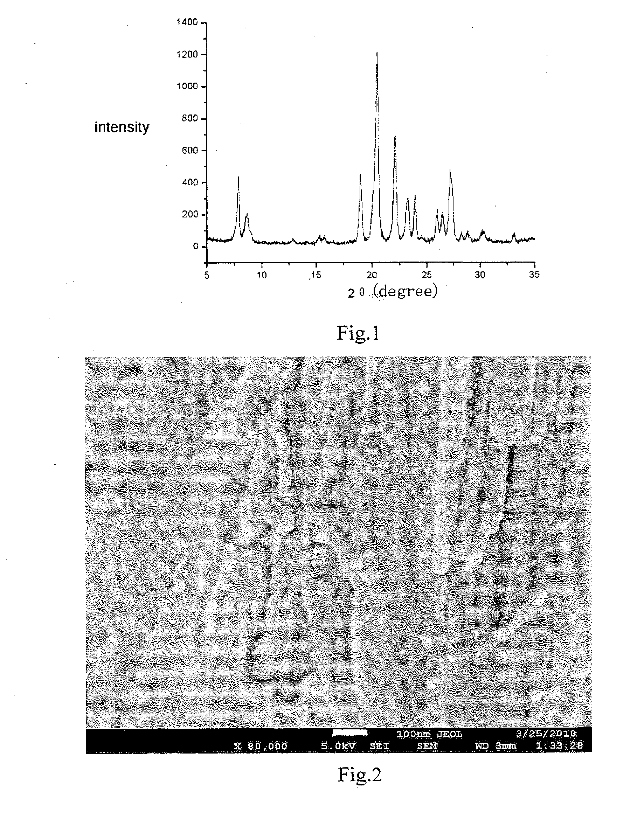

The resultant gel was fed into 1 L synthesis kettle, crystallized at 100 °C and 450 rpm for 1 day, then crystallized at 190°C and 150 rpm for 4 days. Finally, it was cooled down to room temperature, filtered, washed, and dried to obtain the molecular sieves product. Crystalline phase analyses indicated that the product was NU-85 molecular sieves. XRD patterns and the scanning electron microscope spectrum of the product are shown in Fig.1 and 2.

The resultant NU-85 molecular sieve had a specific surface area of 405 m²/g, and a pore volume of 0.35 cm³/g.

### Example 2

Formulating solution 1: 3.73 g of NaAlO₂, 6.48 g of NaOH, 3.82 g of NaCl and 44.67 g of DecBr₂ were added into 350 ml of H₂O, and homogeneously mixed with stirring.

Formulating solution 2: 125 ml of silica sol was added into 250 ml of H₂O, and homogeneously mixed with stirring.

The solution 2 was added into the solution 1, and homogeneously mixed with stirring. 375 g of EU-1 molecular sieve was added as the crystal seed to prepare a gel.

The resultant gel was fed into 1 L synthesis kettle, crystallized at 120°C and 450 rpm for 1 day, then crystallized at 180°C and 100 rpm for 5 days. Finally, it was cooled down to room temperature, filtered, washed, and dried to obtain the molecular sieve product. Crystalline phase analyses indicated that the product was NU-85 molecular sieves.

The resultant NU-85 molecular sieve had a specific surface area of 470 m²/g, and a pore volume of 0.29 cm³/g.

### Example 3

Formulating solution 1: 2.98 g of NaAlO₂, 8.96 g of NaOH, 3.96 g of NaCl and 55.76 g of DecBr₂ were added into 400 ml of H₂O, and homogeneously mixed with stirring.

Formulating solution 2: 125 ml of silica sol was added into 342 ml of H₂O, and homogeneously mixed with stirring.

The solution 2 was added into the solution 1, and homogeneously mixed with stirring. 268 g of EU-1 molecular sieve was added as the crystal seed to prepare a gel.

The resultant gel was fed into 1 L synthesis kettle, crystallized at 160°C and 750 rpm for 1 day, and then crystallized at 170°C and 50 rpm for 4 days. Finally, it was cooled down to room temperature, filtered, washed, and dried to obtain the molecular sieve product. Crystalline phase analyses indicated that the product was NU-85 molecular sieves.

The resultant NU-85 molecular sieve had a specific surface area of 432 m²/g, and a pore volume of 0.31 cm³/g.

### Example 4

Formulating solution 1: 3.25 g of NaAlO₂, 7.23 g of NaOH, 2.13 g of NaCl and 34.89 g of DecBr₂ were added into 350 ml of H₂O, and homogeneously mixed with stirring.

Formulating solution 2: 125 ml of silica sol was added into 250 ml of H₂O, and homogeneously mixed with stirring.

The solution 2 was added into the solution 1, and homogeneously mixed with stirring. 312 g of EU-1 molecular sieve was added as the crystal seed to prepare a gel.

The resultant gel was fed into 1 L synthesis kettle, crystallized at 140°C and 650 rpm for 2 days, and then crystallized at 170°C and 100 rpm for 5 days. Finally, it was cooled down to room temperature, filtered, washed, and dried to obtain the molecular sieve product. Crystalline phase analyses indicated that the product was NU-85 molecular sieves.

The resultant NU-85 molecular sieve had a specific surface area of 428 m²/g, and a pore volume of 0.33 cm³/g.

### Example 5

Formulating solution 1: 3.73 g of NaAlO₂, 8.98 g of NaOH, 4.02 g of NaCl and 36.54 g of DecBr₂ were added into 350 ml of H₂O and homogeneously mixed with stirring.

Formulating solution 2: 125 ml of silica sol was added into 280 ml of H₂O,and homogeneously mixed with stirring.

The solution 2 was added into the solution 1, and homogeneously mixed with stirring. 345 g of EU-1 molecular sieve was added as the crystal seed to prepare a gel.

The resultant gel was fed into 1 L synthesis kettle, crystallized at 160°C and 350 rpm for 1 day, and then crystallized at 190°C and 150 rpm for 3 days. Finally, it was cooled down to room temperature, filtered, washed, and dried to obtain the molecular sieve product. Crystalline phase analyses indicated that the product was NU-85 molecular sieves.

The resultant NU-85 molecular sieve had a specific surface area of 457 m²/g, and a pore volume of 0.27 cm³/g.

### Example 6

Formulating solution 1: 2.89 g of NaAlO₂, 6.36 g of NaOH, 3.94 g of NaCl and 69.01 g of DecBr₂ were added into 400 ml of H₂O, and homogeneously mixed with stirring.

Formulating solution 2: 125 ml of silica sol was added into 250 ml of H₂O, and homogeneously mixed with stirring.

The solution 2 was added into the solution 1, and homogeneously mixed with stirring. 335 g of EU-1 molecular sieve was added as the crystal seed to prepare a gel.

The resultant gel was fed into 1 L synthesis kettle, crystallized at 100°C and 450 rpm for 1 day, then and crystallized at 185 °C and 150 rpm for 4 days. Finally, it was cooled down to room temperature, filtered, washed, and dried to obtain the molecular sieve product. Crystalline phase analyses indicated that the product was NU-85 molecular sieves.

The resultant NU-85 molecular sieve had a specific surface area of 463 m²/g, and a pore volume of 0.28 cm³/g.

### Example 7

Formulating solution 1: 2.88 g of NaAlO₂, 7.63 g of NaOH, 2.13 g of NaCl and 45.13 g of DecBr₂ were added into 350 ml of H₂O, and homogeneously mixed with stirring.

Formulating solution 2: 125 ml of silica sol was added into 250 ml of H₂O, and homogeneously mixed with stirring.

The solution 2 was added into the solution 1, and homogeneously mixed with stirring. 364 g of EU-1 molecular sieve was added as the crystal seed to prepare a gel.

The resultant gel was fed into 1L of a synthesis kettle, crystallized at 140°C and 750 rpm for 1.5 days, and then crystallized at 175°C and 150 rpm for 6 days. Finally, it was cooled down to room temperature, filtered, washed, and dried to obtain the molecular sieve product. Crystalline phase analyses indicated that the product was NU-85 molecular sieves.

The resultant NU-85 molecular sieve had a specific surface area of 464 m²/g, and a pore volume of 0.29 cm³/g.

### Example 8

Formulating solution 1: 4.25 g of NaAlO₂, 9.34 g of NaOH, 4.26 g of NaCl and 25.09 g of DecBr₂ were added into 400 ml of H₂O, and homogeneously mixed with stirring.

Formulating solution 2: 125 ml of silica sol was added into 340 ml of H₂O, and homogeneously mixed with stirring.

The solution 2 was added into the solution 1, and homogeneously mixed with stirring. 364 g of EU-1 molecular sieve was added as the crystal seed to prepare a gel.

The resultant gel was fed into 1L of a synthesis kettle, crystallized at 100°C and 300 rpm for 2 days, and then crystallized at 170°C and 40 rpm for 5 days. Finally, it was cooled down to room temperature, filtered, washed, and dried to obtain the molecular sieve product. Crystalline phase analyses indicated that the product was NU-85 molecular sieves.

The resultant NU-85 molecular sieve had a specific surface area of 453 m²/g, and a pore volume of 0.30 cm³/g.

### Example 9 (Comparative example)

Formulating solution 1: 6.8 g of NaAlO₂, 8.86 g of NaOH, 2.0 g of NaCl and 51.72 g of HexBr₂ (hexamethonium bromide) were added into 350 ml of H₂O, and homogeneously mixed with stirring.

Formulating solution 2: 143 ml of silica sol was added into 290 ml of H₂O, and homogeneously mixed with stirring.

The solution 2 was added into the solution 1 and homogeneously mixed with stirring to prepare a gel having a molar composition of 60 SiO₂:1.7 Al₂O₃:10 Na₂O:10 HexBr₂: 3000 H₂O.

The resultant gel was fed into 1 L synthesis kettle, crystallized at 160°C and 350 rpm for 11 days, and cooled down to room temperature. Finally, it was filtered, washed, dried to obtain the molecular sieve product. Crystalline phase analyses indicated that the product was NU-85 molecular sieves

The resultant NU-85 molecular sieve had a specific surface area of 364 m²/g, and a pore volume of 0.19 cm³/g.

### Example 10

NU-85 molecular sieves prepared in Example 1 were calcined at 550°C for 6 h to remove the template. Then the molecular sieves were mixed with 1M of ammonium nitrate solution in a solid to liquid ratio of 50 g/L, to conduct the ammonium exchange twice at 70°C for 5 h. The mixture was calcined at 550°C for 6 h to obtain the hydrogen form of NU-85 molecular sieve NUZ-1.

The NU-85 molecular sieves prepared in Example 9 (comparative example) were treated under the same conditions to obtain NUZ-2.

### Example 11

Preparation of cumene from benzene and propylene using NU-85 molecular sieves.

Cumene was prepared from benzene and propylene by using NUZ-1 and NUZ-2 obtained in Example 10. The reaction conditions were as follows: a temperature of 110°C, a pressure of 3.0 MPa, a propylene volume space velocity of 15.0 h⁻¹, a benzene/propylene molar ratio of 4. The reaction results were listed in Table 1.

**Table 1 Results of reaction of benzene with propylene**

| Molecular sieves | conversion rate of propylene % | selectivity of cumene % |
|---|---|---|
| NUZ-1 | 96.2 | 89.1 |
| NUZ-2 | 90.5 | 81.8 |

Conversion rate of propylene %= (the molar amount of propylene in the feed stream - the molar amount of propylene in the product stream)/the molar amount of propylene in the feed stream* 100%; Selectivity of cumene %=the molar amount of cumene/the molar amount of the converted propylene* 100%.

### Example 12

C8 aromatic isomerization with using NU-85 molecular sieves.

Alumina, NUZ-1 molecular sieve prepared in Example 10 and an active metal (such as platinum) were mixed, kneaded, extrusion-molded to prepare the catalyst C1. EU-1 molecular sieves were used to prepare the catalyst C2 as the control. The catalyst consisted of 30 wt.% of molecular sieves, 0.3 wt.% of platinum and 69.7 wt.% of alumina. The reaction materials consisted of 0.01 mol.% of benzene, 0.13 mol.% of toluene, 12.16 mol.% of ethylbenzene, 0.4 mol.% of p-xylene, 55.23 mol.% of m-xylene, 22.58 mol.% of o-xylene. The reactions were carried out at a temperature of 395°C, a pressure of 1.0 MPa, a volume space velocity of 4.0 h⁻¹, a hydrogen/hydrocarbon molar ratio of 4.0. The reaction results are listed in Table 2 (mole percentage).

**Table 2 Results of C8 aromatic isomerization**

| Catalysts | PX/ΣX | loss% of C8 aromatic | conversion rate of ethylbenzene % |
|---|---|---|---|
| C1 | 23.5 | 1.7 | 22.2 |
| C2 | 23.1 | 3.9 | 19.1 |

PX/ΣX represents the molar amount of p-xylene/the molar amount of xylenes. loss% of C8 aromatic=(the molar amount of C8 aromatic in the feed stream - the molar amount of C8 aromatic in the product stream)/the molar amount of C8 aromatic in the feed stream* 100%; Conversion rate of ethylbenzene %=(the molar amount of ethylbenzene in the feed stream - the molar amount of ethylbenzene in the product stream)/the molar amount of ethylbenzene in the feed stream* 100%.

### Example 13

Toluene disproprotionation and trimethylbenzene trans-alkylation reaction with using NU-85 molecular sieves.

NUZ-1 molecular sieves prepared in Example 10 were used and Mordenite (MOR) (prepared according to the process of Example 9 in CN 1230518) was used as the comparative molecular sieve. The raw materials consisted of 60% toluene and 40% 1,2,4-trimethylbenzene. The reaction conditions were as follows: a temperature of 400 °C, a pressure of 2.4 MPa, a volume space velocity of 1.2 h⁻¹, a hydrogen/hydrocarbon volume ratio of 13. The reaction results are listed in Table 3 (mole percentage).

**Table 3 Results of toluene disproprotionation and trimethylbenzene trans-alkylation reaction**

| Molecular sieves | conversion rate of Toluene% | Conversion rate of trimethylbenzene conversion rate % | Total selectivity of benzene and C₈ aromatic % | Selectivity of xylene % |
|---|---|---|---|---|
| NUZ-1 | 41.5 | 48.3 | 93.8 | 87.5 |
| MOR | 37.8 | 44.6 | 93.1 | 84.4 |

Conversion rate of toluene %=(the molar amount of toluene in the feed stream - the molar amount of toluene in the product stream)/the molar amount of toluene in the feed stream* 100%; Conversion rate of trimethylbenzene %=(the molar amount of trimethylbenzene in the feed stream - the molar amount of trimethylbenzene in the product stream)/the molar amount of trimethylbenzene in the feed stream* 100%; Total selectivity of benzene and C8 aromatic %=(the molar amount of benzene in the product + the molar amount of C8 aromatic in the product)/(the molar amount of the converted toluene and the converted trimethylbenzene)* 100%; Selectivity of xylene %=the molar amount of xylene in the product/(the molar amount of the converted toluene and the converted trimethylbenzene)* 100%.

### Example 14

Preparing ethylbenzene using benzene and ethylene with using NU-85 molecular sieves.

NUZ-1 molecular sieves prepared in Example 10 were used, and β zeolites (prepared according to the process of Example 1 in CN1362361) were used as the comparative molecular sieves. The reaction conditions were as follows: a temperature of 220°C, a pressure of 3.5 MPa, an ethylene volume space velocity of 2.0 h⁻¹, and starting material of benzene/ethylene molar ratio of 12. The reaction results are listed in Table 4 (mole percentage).

**Table 4 Results of reaction of benzene with ethylene**

| Molecular sieves | conversion rate of ethylene % | selectivity of ethylbenzene % |
|---|---|---|
| NUZ-1 | 56.4 | 95.5 |
| β zeolite | 41.2 | 90.7 |

Conversion rate of propylene %= (the molar amount of propylene in the feed stream - the molar amount of ethylene in the product stream)/the molar amount of ethylene in the feed stream* 100%; selectivity of ethylbenzene %=the molar amount of ethylbenzene /the molar amount of the converted ethylene* 100%.

## Claims

1. A NU-85 molecular sieve, wherein it has a specific surface area ranging from 405 m²/g to 470 m²/g, and a pore volume ranging from 0.27 cm³/g to 0.35 cm³/g.

2. The NU-85 molecular sieve according to claim 1, wherein it has a specific surface area ranging from 410 m²/g to 460 m²/g.

3. The NU-85 molecular sieve according to claim 1 or 2, wherein it has a pore volume ranging from 0.28 cm³/g to 0.32 cm³/g.

4. A process for preparing the NU-85 molecular sieve according to claim 1, comprising: mixing an aluminum source, a silicon source, a base, a template, an inorganic salt, EU-1 molecular sieve and water in a mass ratio of the EU-1 molecular sieve to SiO₂ in the silicon source ranging from 2.5 to 8.5 to formulate crystallization material, and a molar ratio of the aluminum source (calculated based on Al₂O₃), silicon source (calculated based on SiO₂), base (calculated based on Na₂O), template, inorganic salt (calculated based on NaL) and water in the crystallization material ranging as follows:
SiO₂/Al₂O₃= 30:1 to 70:1;
R/SiO₂= 0.04:1 to 0.25:1;
Na₂O/SiO₂= 0.05:1 to 0.3:1;
H₂O/SiO₂= 35:1 to 80:1;
NaL/SiO₂= 0.03:1 to 0.14:1;
wherein R is a template selected from the group consisting of nonamethonium bromide, decamethonium bromide, and undecamethonium bromide, and mixture thereof; NaL is a sodium salt; and
crystallizing the formulated crystallization material by dynamic crystallizing procedure comprising two stages: low-temperature crystallization and high-temperatrue crystallization, wherein the low-temperature crystallization is firstly carried out by high-speed stirring at a stirring rate ranging from 300rpm to 800 rpm and at a crystallization temperature ranging from 100°C to 160°C for a period of time ranging from 1 day to 2 days; then the high-temperature crystallization is carried out by low-speed stirring at a stirring rate ranging from 40 rpm to 250 rpm and at a crystallization temperature ranging from 170°C to 190°C for a period of time ranging from 2 days to 6 days.

5. The process according to claim 4, wherein the molar ratio of the aluminum source, silicon source, the base, the template, the inorganic salt and water in the crystallization material range as follows
SiO₂/Al₂O₃= 40:1 to 65:1;
R/SiO₂= 0.08:1 to 0.22:1;
Na₂O/SiO₂= 0.1:1 to 0.2:1;
H₂O/SiO₂= 42:1 to 55:1; and
NaL/SiO₂= 0.0049:1 to 0.098:1.

6. The process according to claim 4 or 5, wherein the mass ratio of the EU-1 molecular sieve to SiO₂ in the silicon source is from 3.0:1 to 7.0:1.

7. The process according to any one of the claims 4 to 6, wherein the crystallization pressure during the entire crystallizing procedure is carried out under autogenous pressure at said crystallization temperature.

8. The process according to any one of the claims 4 to 7, wherein the sodium salt NaL is selected from the group consisting of sodium chloride, sodium bromide, sodium nitrate, sodium acetate and sodium oxalate, and mixture thereof.

9. The process according to any one of the claims 4 to 8, wherein the aluminum source is one selected from the group consisting of aluminum salts, aluminates, alumina, aluminum hydroxide and alumina sols, and mixtures thereof.

10. The process according to any one of the claims 4 to 9, wherein the silicon source is selected from the group consisting of white carbon black, silica sol, water glass and tetraethyl orthosilicate, and mixtures thereof.

11. The process according to any one of the claims 4 to 10, wherein the SiO₂/Al₂O₃ molar ratio in the EU-1 molecular sieve ranges from 20:1 to 35:1.

12. The use of the NU-85 molecular sieve according to any one of the claims 1 to 3 in aromatic alkylation, aromatic isomerization and alkane hydroisomerization.

## Patentansprüche

1. NU-85-Molekularsieb, das eine spezifische Oberfläche im Bereich von 405 - 470 m²/g und ein Porenvolumen im Bereich von 0,27 - 0,35 cm³/g hat.

2. NU-85-Molekularsieb nach Anspruch 1, das eine spezifische Oberfläche im Bereich von 410 - 460 m²/g hat.

3. NU-85-Molekularsieb nach Anspruch 1 oder 2, das ein Porenvolumen im Bereich von 0,28 - 0,32 cm³/g hat.

4. Verfahren zur Erzeugung des NU-85-Molekularsiebes nach Anspruch 1, umfassend: Mischen einer Aluminiumquelle, einer Siliziumquelle, einer Basis, eines Templates, eines anorganischen Salzes, EU-1-Molekularsieb und Wasser in einem Massenverhältnis von dem EU-1-Molekularsieb zu SiO₂ in der Siliziumquelle im Bereich von 2,5 bis 8,5, zum Formulieren eines Kristallisationsmaterials, und worin ein molares Verhältnis der Aluminiumquelle (berechnet auf der Basis von Al₂O₃), der Siliziumquelle (berechnet auf der Basis von SiO₂), der Basis (berechnet auf Na₂O), des Templates, des anorganischen Salzes (berechnet auf der Basis von NaL) und Wasser im Kristallisationsmaterial im Bereich wie folgt liegt:
SiO₂/Al₂O₃ = 30:1 bis 70:1;
R/SiO₂ = 0,04:1 bis 0,25:1;
Na₂O/SiO₂ = 0,05:1 bis 0,3:1;
H₂O/SiO₂ = 35:1 bis 80:1;
NaL/SiO₂ = 0,03:1 bis 0,14:1;
worin R ein Template ist, ausgewählt aus der Gruppe bestehend aus Nonamethoniumbromid, Decamethoniumbromid und Undecamethoniumbromid und einer Mischung davon; NaL ein Natriumsalz ist; und
Kristallisieren des formulierten Kristallisationsmaterials durch einen dynamischen Kristallisationsvorgang, umfassend zwei Stufen: Niedertemperaturkristallisierung und Hochtemperaturkristallisierung, worin die Niedertemperaturkristallisierung zunächst durchgeführt wird durch Hochgeschwindigkeitsrühren bei einer Rührrate im Bereich von 300 - 800 Upm und bei einer Kristallisationstemperatur im Bereich von 100 - 160°C für eine Zeitperiode im Bereich von 1 Tag bis 2 Tagen; anschließendes Durchführen der Hochtemperaturkristallisierung durch Niedergeschwindigkeitsrühren bei einer Rührrate im Bereich von 40 - 250 Upm und einer Kristallisationstemperatur im Bereich von 170 - 190°C für eine Zeitperiode im Bereich von 2 - 6 Tagen.

5. Verfahren nach Anspruch 4, worin das molare Verhältnis der Aluminiumquelle, Siliziumquelle, der Basis, des Templates, des anorganischen Salzes und Wasser im Kristallisationsmaterial im Bereich wie folgt liegt:
SiO₂/Al₂O₃ = 40:1 bis 65:1;
R/SiO₂ = 0,08:1 bis 0,22:1;
Na₂O/SiO₂ = 0,1:1 bis 0,2:1;
H₂O/SiO₂ = 42:1 bis 55:1 und
NaL/SiO₂ = 0,0049:1 bis 0,098:1.

6. Verfahren nach Anspruch 4 oder 5, worin das Massenverhältnis des EU-1-Molekularsiebes zu SiO₂ in der Siliziumquelle von 3,0:1 bis 7,0:1 ist.

7. Verfahren nach einem der Ansprüche 4 bis 6, worin der Kristallisationsdruck während des vollständigen Kristallisationsvorgangs unter autogenem Druck bei der Kristallisationstemperatur durchgeführt wird.

8. Verfahren nach einem der Ansprüche 4 bis 7, worin das Natriumsalz NaL ausgewählt ist aus der Gruppe, bestehend aus Natriumchlorid, Natriumbromid, Natriumnitrat, Natriumacetat und Natriumoxalat und Mischungen davon.

9. Verfahren nach einem der Ansprüche 4 bis 8, worin die Aluminiumquelle eine ist, ausgewählt aus der Gruppe bestehend aus Aluminiumsalzen, Aluminaten, Alumina, Aluminiumhydroxid und Aluminasolen und Mischungen davon.

10. Verfahren nach einem der Ansprüche 4 bis 9, worin die Siliziumquelle ausgewählt ist aus der Gruppe bestehend aus weißem Ruß, Silicasol, Wasserglas und Tetraethylorthosilicat und Mischungen davon.

11. Verfahren nach einem der Ansprüche 4 bis 10, worin das molare SiO₂/Al₂O₃-Verhältnis im EU-1-Molekularsieb im Bereich von 20:1 bis 35:1 liegt.

12. Verwendung des NU-85-Molekularsiebes nach einem der Ansprüche 1 bis 3 bei der aromatischen Alkylierung, aromatischen Isomerisierung und Alkanhydroisomerisierung.

## Revendications

1. Tamis moléculaire NU-85, **caractérisé en ce qu'**il présente une surface spécifique comprise entre 405 m²/g et 470 m²/g, et un volume de pores compris entre 0,27 cm³/g et 0,35 cm³/g.

2. Tamis moléculaire NU-85 selon la revendication 1, **caractérisé en ce qu'**il présente une surface spécifique comprise entre 410 m²/g et 460 m²/g.

3. Tamis moléculaire NU-85 selon la revendication 1 ou 2, **caractérisé en ce qu'**il présente un volume de pores compris entre 0,28 cm³/g et 0,32 cm³/g.

4. Procédé de préparation du tamis moléculaire NU-85 selon la revendication 1, comprenant : le mélange d'une source d'aluminium, d'une source de silicium, d'une base, d'une matrice, d'un sel inorganique, d'un tamis moléculaire EU-1 et d'eau dans un rapport de masses du tamis moléculaire UE-1 au SiO₂ dans la source de silicium compris entre 2,5 et 8,5 pour formuler un matériau de cristallisation, et un rapport molaire de la source d'aluminium (calculé sur la base de l'Al₂O₃), la source de silicium (calculé sur la base du SiO₂), la base (calculé sur la base du Na₂O), la matrice, du sel inorganique (calculé sur la base du NaL) et l'eau dans le matériau de cristallisation variant comme suit :
SiO₂/Al₂O₃ = 30:1 à 70:1 ;
R/SiO₂ = 0,04:1 à 0,25:1 ;
Na₂O/SiO₂ = 0,05:1 à 0,3:1 ;
H₂O/SiO₂ = 35:1 à 80:1 ;
NaL/SiO₂ = 0,03:1 à 0,14:1 ;
dans lequel R est une matrice choisie dans le groupe constitué par le bromure de nonaméthonium, le bromure de décaméthonium, et le bromure d'undécaméthonium, et leurs mélanges ; NaL est un sel de sodium ; et
la cristallisation du matériau de cristallisation formulé par une procédure de cristallisation dynamique comprenant deux étapes : une cristallisation à basse température et une cristallisation à haute température, dans laquelle la cristallisation à basse température est tout d'abord réalisée par agitation à grande vitesse à une vitesse d'agitation comprise entre 300 tours par minutes et 800 tours par minute et à une température de cristallisation comprise entre 100°C et 160°C pendant une période comprise entre 1 jour et 2 jours ; puis la cristallisation à haute température est réalisée par agitation à basse vitesse à une vitesse d'agitation comprise entre 40 tours par minute et 250 tours par minute et à une température de cristallisation comprise entre 170°C et 190°C pendant une période comprise entre 2 jours et 6 jours.

5. Procédé selon la revendication 4, dans lequel le rapport molaire de la source d'aluminium, la source de silicium, la base, la matrice, le sel inorganique et l'eau dans le matériau de cristallisation varie comme suit
SiO₂/Al₂O₃ = 40:1 à 65:1,
R/SiO₂ = 0,08:1 à 0,22:1 ;
Na₂O/SiO₂ = 0,1:1 à 0,2:1 ;
H₂O/SiO₂ = 42:1 à 55:1 ; et
NaL/SiO₂ = 0,0049:1 à 0,098:1.

6. Procédé selon la revendication 4 ou 5, dans lequel le rapport de masses du tamis moléculaire EU-1 à SiO₂ dans la source de silicium est de 3,0:1 à 7,0:1.

7. Procédé selon l'une quelconque des revendications 4 à 6, dans lequel la pression de cristallisation au cours de la procédure entière de cristallisation est réalisée sous pression autogène, à ladite température de cristallisation.

8. Procédé selon l'une quelconque des revendications 4 à 7, dans lequel le sel de sodium NaL est choisi dans le groupe constitué par le chlorure de sodium, le bromure de sodium, le nitrate de sodium, l'acétate de sodium et l'oxalate de sodium, et leurs mélanges.

9. Procédé selon l'une quelconque des revendications 4 à 8, dans lequel la source d'aluminium est un élément choisi dans le groupe constitué par les sels d'aluminium, les aluminates, l'alumine, l'hydroxyde d'aluminium et les sols d'alumine, et leurs mélanges.

10. Procédé selon l'une quelconque des revendications 4 à 9, dans lequel la source de silicium est choisie dans le groupe constitué par le noir de carbone blanc, un sol de silice, un verre soluble et l'orthosilicate de tétraéthyle, et leurs mélanges.

11. Procédé selon l'une quelconque des revendications 4 à 10, dans lequel le rapport molaire de SiO₂/Al₂O₃ dans le tamis moléculaire EU-1 est compris entre 20:1 et 35:1.

12. Utilisation du tamis moléculaire NU-85 selon l'une quelconque des revendications 1 à 3 dans une alkylation de composé aromatique, une isomérisation de composé aromatique et une hydroisomérisation d'alcane.
